# EUROPEAN PATENT SPECIFICATION

(11) **EP 4 228 760 B1**
(45) Date of publication and mention of the grant of the patent: **02.12.2026**
(21) Application number: 21881227.9
(22) Date of filing: 15.10.2021
(51) Int. Cl.: A61K 31/4375, A61K 31/5377, A61K 45/06, A61K 9/00, A61K 9/08, A61K 47/02, A61K 47/10, A61K 47/20, A61P 21/00, A61P 21/02, A61P 23/00, C07D 405/12

(54) **DOSAGE REGIMENS FOR RP1000 AND RP2000 FOR USE IN A METHOD OF INDUCING PARALYSIS OR NEUROMUSCULAR BLOCKADE AND RECOVERY THEREFROM**
DOSIERUNGSSCHEMEN FÜR RP1000 UND RP2000 ZUR ANWENDUNG BEI EINEM VERFAHREN ZUR EINLÖSUNG VON PARALYSE ODER NEUROMUSKULÄRER BLOCKADE UND DEREN ERHOLUNG
POSOLOGIE DU RP1000 ET DU RP2000 UTILISÉS DANS UNE MÉTHODE D'INDUCTION DE LA PARALYSIE OU DU BLOCAGE NEUROMUSCULAIRE ET DE LA RÉCUPÉRATION QUI EN DÉCOULE

(30) Priority: 17.10.2020 US 202063093179 P
(43) Date of publication of application: 23.08.2023
(73) Proprietor: Cornell University, Ithaca, NY 14850 (US)
(72) Inventor: SAVARESE, John J., Southbury, Connecticut 06488 (US)
(74) Representative: Secerna LLP
(86) International application number: PCT/US2021/055289
(87) International publication number: WO 2022/082051

(56) References cited:
- US-A1- 2006 217 628
- US-A1- 2015 191 453
- US-A1- 2020 024 301
- SUNAGA HIROSHI ET AL: "Preclinical Pharmacology of CW002", ANESTHESIOLOGY, vol. 125, no. 4, 1 October 2016 (2016-10-01), US, pages 732 - 743, XP093191173, ISSN: 0003-3022, DOI: 10.1097/ALN.0000000000001254
- HEERDT PAUL M. ET AL: "Novel neuromuscular blocking drugs and antagonists", CURRENT OPINION IN ANAESTHESIOLOGY., vol. 28, no. 4, 1 August 2015 (2015-08-01), US, pages 403 - 410, XP093191178, ISSN: 0952-7907, DOI: 10.1097/ACO.0000000000000209
- HUNTER JENNIFER M: "Neuromuscular blocking agents and reversal agents", ANAESTHESIA, ELSEVIER, AMSTERDAM, NL, vol. 22, no. 6, 24 May 2021 (2021-05-24), pages 380 - 384, XP086600971, ISSN: 1472-0299, [retrieved on 20210524], DOI: 10.1016/J.MPAIC.2021.04.010
- SAVARESE JOHN J., SUNAGA HIROSHI, MCGILVRA JEFF D., BELMONT MATTHEW R., MURRELL MATTHEW T., JEANNOTTE ERIN, COOKE FARRELL E., WAST: "Preclinical Pharmacology in the Rhesus Monkey of CW 1759-50, a New Ultra- short Acting Nondepolarizing Neuromuscular Blocking Agent, Degraded and Antagonized by L- Cysieine", ANESTHESIOLOGY, vol. 129, no. 5, November 2018 (2018-11-01), pages 970 - 988, XP055933188

## Description

### TECHNICAL FIELD

The present disclosure relates to neuromuscular blockade agents (NMBAs) and more specifically to methods for predicting and controlling spontaneous patient recovery after administration of the NMBA to the patient.

### BACKGROUND

Neuromuscular blockade (NMB) is frequently used in anesthesia to facilitate endotracheal intubation, optimize surgical conditions, and assist with mechanical ventilation in patients who have reduced lung compliance. To avoid the postoperative residual effects of NMBAs, full metabolism of the NMBA into inactive metabolites should be fully achieved prior to extubation. As such, it is common (though not universal) practice to closely monitored the depth of paralysis as an indirect measure of active NMBA in the patient's system. Depth of paralysis may be monitored by available neuromuscular stimulating techniques such as train-of-four (TOF), single twitch (ST), double burst (DBS) and post-tetanic count (PTC).

The most commonly used neuromuscular sensing modality is the TOF measurement by electrostimulation. TOF typically uses four brief (between 100 and 300 µs) current pulses (generally less than 70 mA) at 2 Hz, repeated every 10 to 20 s as electrostimulation. The resulting twitches are measured and quantified for electromyographic response, force, acceleration, deflection or another means. The first-the T1 twitch, and the last-the T4 twitch, are compared, and the ratio of the two (TOFR) gives an estimate of the level of NMB. Stimuli series are spaced by ten or more seconds (generally 20 s is used to provide a margin of safety) to give a rest period for full restoration of steady state conditions-faster stimulation results in smaller evoked responses. Other methods for monitoring extent of NMB include the single twitch (ST) measurement, double burst stimulation (DBS), and post-tetanic count (PTC).

However, even with close monitoring, the use of NMBAs, (particularly those characterized as longer acting) still frequently leads to residual paralyzing effects in the postoperative period (postoperative residual curarization (PORC)) due to incomplete transformation the administered paralyzing agent into its inactive form at the level of neuromuscular junctions. The safety of NMBAs is highly scrutinized, debated, and of utmost importance. Incomplete recovery from NMBAs (residual block) after anesthesia and surgery continues to be a common problem in the post-anesthesia care unit and pose a threat to patient safety. Adverse effects of residual block include, but are not limited to, airway obstruction, hypoxemic episodes, postoperative respiratory complications, intraoperative awareness, and unpleasant symptoms of muscle weakness.

The reversal of the NMB may be achieved with reversal agents, however, the most common NMBA reversal agent, acetylcholinesterase inhibitor (AChEI), simply antagonizes the paralyzing agent. It does not hasten NMBA metabolism. As such, even with the use of NMBA reversal agents, a residual curarization may still occur as the body metabolizes the reversal agent in normal course. Additionally, current practice dictates that an anesthesiologist must wait until a patient is spontaneously beginning to recover from NMBA before administering an antagonist. Often, this waiting time ranges from 30 to 60 minutes or more.

Prediction and/or control of NMB recovery may be derived from agency guidelines. For example, the FDA mandates a maximum allowed clinical duration of an NMBA, measured as the time for return to a twitch height 25% above baseline in a twitch response test after administration of a dose twice the 95% effective dose (ED₉₅).

What is needed is a simple method for inducing as well as effecting recovery from NMB in a patient that is effective and reduces incidence of post-operative residual effects. Since compliance with intra-operative monitoring is not universal and not always possible, the field would benefit from a method providing highly predictable NMBA recovery periods - both in timing as well as degree of recovery. Described herein below is one such method.

US 2015/191453 discloses the induction of neuromuscular blocking by RP1000 (referred to as CW 002) and RP2000 (referred to as 1759-50).

*Anesthesiology2018,* **129,** 970 discloses the induction of neuromuscular blocking by RP2000 (referred to in as CW 1759-50).

*Anesthesiology2016,* **125,** 732 discloses the pre-clinical pharmacology of RP1000 (referred to as CW002) as an inducer of neuromuscular blocking in the rhesus monkey and cat.

*Curr. Opin. Anaesthesiol.* 2015, **28,** 403 discloses that RP2000 (referred to as CW002) is an intermediate duration, non-depolarizing, cysteine-inactivated, neuromuscular blocking drug which can be reversed at any time by cysteine injection.

*Anesthesiology2016,* **125,** 1136 discloses the dose-response and cardiopulmonary side effects of CW002 (RP1000) in man, and states that CW-002 has a neuromuscular-blocking action.

### SUMMARY

The invention is as disclosed in the appended claims. In particular, the disclosure relates to a compound for use in a method for inducing paralysis or neuromuscular blockade (NMB) and recovery therefrom as described in the appended claims. The method has highly predictable NMBA recovery periods - both in timing as well as degree of recovery.

### BRIEF DESCRIPTION OF THE DRAWINGS

Figure 1 is a graphic representation of the twitch height v. recovery intervals.
Figure 2 is a graphic representation of the twitch height v. recovery intervals.
Figure 3 illustrates the recovery curves of CW002 in healthy adult volunteers under sevoflurane/N2O anesthesia. The curves illustrate spontaneous recovery after 100 percent block, from 5% T1 to 95% of baseline T1. Left to right: Groups 0.08mg/kg (n=2); 0.01mg/kg (n=6); and 0.14 mg/kg (n=4). These doses are ~1.0, 1.4 and 1.8 x ED95. Fourth curve to the right is the composite of all three groups (composite curve, n=12). There are no significant differences among the groups in comparisons gone by ANOVA of the 5-95%recovery intervals.
Figure 4 illustrates the linear regression for the composite group (n=12). Times for recovery of T1 from 5% to T1 of 25%, 50%, 75%, and 95% of baseline. The linear relationship is significant (P=0.002). This suggests that rather precise prediction of time required for recovery from CW002-induced NMB may be done in humans.

### DETAILED DESCRIPTION

During a typical medical operative procedure, a patient may be administered various chemical agents to reduce discomfort and/or prevent movement from interfering with the medical procedure. A patient may first be administered an anesthetic agent to induce anesthesia. Anesthesia, as used herein, refers to a state of controlled, temporary loss of sensation or awareness induced for medical purposes (e.g., surgical operations) and may be maintained during an intra-anesthetic period by continuous or intermittent administration of the anesthetic.

Anesthesia may be administered via inhalation or intravenously. Inhaled anesthesia, as used herein and unless otherwise indicated, refers to anesthesia by respiration of a vapors from a volatile liquid or gaseous anesthetic agent into to the respiratory passages and tract of the patient. Suitable inhalants include, but are not limited to, nitrous oxide (N₂O), desflurane, sevoflurane, isoflurane, methyoxyflurane, halothane, and any combination thereof. One of ordinary skill in the art will be familiar with inhaled anesthetics as well as the methods for their use.

Intravenous anesthesia, as used herein and unless otherwise indicated, refers to administration of a liquid anesthetic to a patient's vein or veins. Suitable intravenous anesthetics include, but are not limited to, propofol, etomidate, NMDA antagonists (e.g., ketamine), dexmedetomidine, barbituates (e.g., thiopental and methohexital), synthetic opioids (e.g., remifentanil, sufentanil), benzodiazepines (e.g., midazolam, diazepam, lorazepam) and any combination thereof. One of ordinary skill in the art will be familiar with IV anesthetics as well as the methods for their use.

Once under anesthesia, a patient may be administered an NMBA, if needed, for example, to intubate the patient. An NMBA is typically administered intravenously or intramuscularly.

Administration of an anesthetic and the administration of an NMBA are each accompanied by an onset period which spans the time of first administration of the agent to a later time where the agent has taken full effect. A medical procedure, e.g., a surgical operation, may be carried out during an intra-operative period after the anesthetic and NMBA have taken full effect. After the medical procedure has finished, administration of NMBA and anesthesia may be discontinued to effect recovery therefrom. Similar to the onset period, discontinuation of anesthetic and NMB agents are accompanied by a recovery period which spans the time where the anesthetic or NMB agent is first discontinued to a later time where the effects of the agent have been fully reversed. At this point of full reversal, recovery is deemed to be achieved.

As used herein, recovery from NMB is considered to be achieved when a TOF ratio (TOFR) of at least about 0.90 is measured. For example, a TOFR of about 0.90 to 1.00 may be measured. Recovery may be achieved with or without the use or administration of an antagonist to the NMBA. As used herein, "spontaneous recovery" is considered to be achieved when a TOFR of at least about 0.90 is measured without the use or administration of an NMBA antagonist. Optionally, other metrics may be used to characterize recovery and/or spontaneous recovery such as, but not limited to, a twitch height of at least 95% over baseline.

RP1000, alternatively called AV002 or CW002, is a non-depolarizing, intermediate-duration NMBA, shown below.

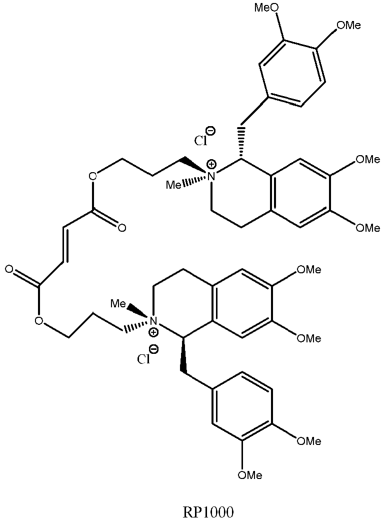

Chemically, RP1000 may be referred to by its IUPAC name, (2S)-1-(3,4-dimethoxybenzyl)-2-(3-(((E)-4-(3-((1R,2S)-1-(3,4-dimethoxybenzyl)-6,7-dimethoxy-2-methyl-1,2,3,4-tetrahydroisoquinolin-2-ium-2-yl)propoxy)-4-oxobut-2-enoyl)oxy)propyl)-6,7-dimethoxy-2-methyl-1,2,3,4-tetrahydroisoquinolin-2-ium dichloride.

The terms "RP1000," "AV002" and "CW002" are used herein interchangeably and refer to the structure above identified as RP1000 herein. Even though RP1000 pictured above reflects the chloride salt form of RP1000, RP1000, as used herein, may also include any other pharmaceutically acceptable and effective salts thereof. RP1000 has been previously disclosed in US Patent 8,148,398, and Prabhakar, et al. (Journal of Anesthesiology and Clinical Pharmacology, 2016 Jul-Sep; 32(3): 376-378), both of which are referenced herein with respect to its disclosure of the RP1000 (or AV002) compound, methods of its preparation, its formulations, as well as methods of use. Preclinical studies with RP1000 have demonstrated 100% NMB within about 90 seconds of administration.

Another non-depolarizing NMBA is RP2000 (alternatively called "CW 1759-50"), which is a short-acting NMBA.

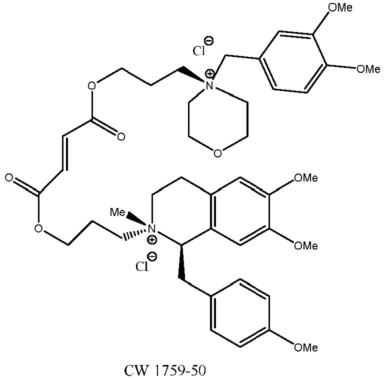

Chemically, RP2000 may be referred to by its IUPAC name 4-(3-(((E)-4-(3-((1R)-6,7-dimethoxy-1-(4-methoxybenzyl)-2-methyl-1,2,3,4-tetrahydro-2-isoquinolin-2-ium-2-yl)propoxy)-4-oxobut-2-enoyl)oxy)propyl)-4-(3,4-dimethoxybenzyl)morpholin-4-ium. The terms "CW 1759-50" and "RP2000" are used herein interchangeably and refer to the structure above identified as RP2000 herein. Even though RP2000 pictured above reflects the chloride salt form of RP2000, RP2000, as used herein, may also include any other pharmaceutically acceptable and effective salts thereof.

The invention is as disclosed in the appended claims. For the avoidance of doubt, the invention provides a compound for use in a method of inducing paralysis or neuromuscular blockade (NMB) and recovery therefrom as described in the appended claims. Therefore, any reference herein to a method of treatment, for example to a method of inducing paralysis or neuromuscular blockade (NMB) and recovery therefrom, is to be interpreted as relating to a compound for use in such a method.

Disclosed herein is a method for inducing and effecting spontaneous recovery from NMB in a patient comprising administering to the patient an effective amount of at least one of RP1000 or RP2000. The method can provide highly predictable NMBA recovery periods - both in timing as well as degree of recovery. Using methods disclosed herein, NMB recovery may be predicted and controlled in relation to one or more other relevant events such as end of the intra-operative period, the recovery period from the anesthetic, and/or achievement of recovery from the anesthetic. Additionally, degree of recovery may be accurately predicted due to a substantially linear correlation between time after administration discontinuation to various measured time points during an NMB recovery period (e.g., 5% twitch, 10% twitch, 25% twitch, 50% twitch, 75% twitch, and 95% twitch (all compared to baseline)). This property of RP1000 can provide methods for inducing NMB during anesthesia with the ability to minimize time spent under NMB post-operatively through accurate prediction of recovery period duration. For example, NMB administration may be discontinued prior to the end of an inter-operative period with the knowledge that the surgical procedures will be finished by the time the patient begins to emerge from NMB blockade.

Therefore, one aspect of the present disclosure provides a method of inducing NMB comprising administering RP1000 to a human patient under inhaled anesthesia in an amount effective to maintain a twitch height of not more than about 5% above a baseline measurement, thereby inducing NMB in the human patient; and, after a desired duration, discontinuing administration of the RP1000 to the patient, thereby effecting a spontaneous recovery of the patient from the NMB. An "effective amount" of a compound is a predetermined amount calculated to achieve the desired effect (e.g., degree of NMB measured by twitch height). An "effective amount" of a compound with respect to use in treatment, refers to an amount of the compound in a preparation which, when administered as part of a desired dosage regimen (to a mammal, such as a human) alleviates a symptom, ameliorates a condition, or slows the onset of disease conditions according to clinically acceptable standards for the disorder or condition to be treated or the cosmetic purpose, e.g., at a reasonable benefit/risk ratio applicable to any medical treatment. Optionally, NMB may be induced during an intra-operative period and/or during an intra-anesthetic period. An "effective amount" of the compound RP1000 and an "effective amount" of the compound RP2000 is as described in the appended claims.

RP1000 may be administered to the human patient in a single dose or in multiple doses, each dose comprising RP1000 in an amount of about 1.0 to about 3.0 times the ED₉₅ for humans (about 0.077 mg/kg). A dose may be administered in a single IV bolus dose, multiple IV bolus doses, or may be administered as a continuous IV infusion. Administration of a single bolus of RP1000 may be carried out over a time period of about 5 seconds to about 15 seconds. Administration in this manner may be continued, as needed, throughout an inter-operative procedure to maintain NMB (not more than about 5% twitch compared to baseline). Alternatively, RP1000 may be administered as a slower infusion over a time period of about 1 minute to about 2 minutes or as a continuous slow infusion spanning, e.g., at least a portion of an intra-operative period.

Specific doses include, but are not limited to, about 0.15 mg (on a cation basis) per kg of body weight to about 0.25 mg/kg RP1000 may be administered to a patient. Other contemplated dosage ranges include about 0.15 mg/kg to about 0.20 mg/kg, about 0.15 mg/kg to about 0.25 mg/kg RP1000. Specific dosages include any there between, such as, but not limited to, 0.16 mg/kg, 0.2 mg/kg, 0.24 mg/kg, and 0.3 mg/kg RP1000. The patient may be under inhaled anesthesia, for example, nitrous oxide, desflurane, sevoflurane, isoflurane, methyoxyflurane, or any combination thereof. The dose of anesthesia may be any desired dose, for example, 0.5 MAC, 0.75 MAC, 1.0 MAC, 1.25 MAC, or higher. At higher anesthesia dosing, spontaneous recovery is still expected to be predictable, albeit longer due to the deepened state of anesthesia.

Additionally, in one or more embodiments, a twitch height of 25% above baseline may be measured in the patient within about 14 minutes, 11 minutes, or 8 minutes after discontinuation of RP1000 administration. In one or more embodiments, a twitch height of 50% above baseline may be measured in the patient within about 28 minutes, about 22 minutes, or within about 17 minutes after discontinuation of RP1000 administration. In one or more embodiments, a twitch height of 75% above baseline may be measured in the patient within about 42 minutes, about 33 minutes, or 25 minutes after discontinuation of RP1000 administration. In various embodiments, the patient is under inhaled anesthesia at a concentration not greater than about 1.5 MACs. In various embodiments, the patient is under inhaled anesthesia at a concentration not greater than about 1.0 MACs.

RP1000 has a relatively rapid onset period, providing an additional opportunity to minimize the time a patient is under NMB. Therefore, optionally and additionally, the administration of RP1000 to a patient during the intra-anesthetic period may effect a measured twitch height in the patient of not more than about 5% above a baseline measurement within about 2 minutes, more preferably within about 90 seconds, after administration begins.

The method as described herein comprises effecting spontaneous recovery from NMB without the use of an antagonist or reversal agent of an NMBA. In one or more embodiments, spontaneous recovery is achieved not more than about 50 minutes after RP1000 administration is discontinued. More preferably, spontaneous recovery is achieved in not more than about 40 minutes, not more than about 30 minutes or not more than about 25 minutes. Additional measurements may supplement the TOFR measurement, such as measuring a twitch height of at least 95% over baseline.

RP2000 can be used as an NMBA. As such, another aspect of the present disclosure provides a method of inducing NMB comprising administering RP2000 to a human patient under inhaled anesthesia in an amount effective to maintain a twitch height of not more than about 5% above a baseline measurement, thereby inducing NMB in the human patient; and, after a desired duration, discontinuing administration of the RP2000 to the patient, thereby effecting a spontaneous recovery of the patient from the NMB. Optionally, NMB may be induced during an intra-operative period and/or during an intra-anesthetic period.

RP2000 may be administered to the human patient in a single dose or in multiple doses, each dose comprising RP2000 in an amount of about 1.0 to about 3.0 times the ED₉₅ for humans (about 0.077 mg/kg). A dose may be administered through multiple IV bolus doses or may be administered as a continuous IV infusion. Administration of a single bolus of RP1000 may be carried out over a time period of about 5 seconds to about 15 seconds. Administration in this manner may be continued, as needed, throughout an inter-operative procedure to maintain NMB (not more than about 5% twitch compared to baseline). Alternatively, RP1000 may be administered as a slower infusion over a time period of about 1 minute to about 2 minutes or as a continuous slow infusion spanning, e.g., at least a portion of an intra-operative period.

Since the duration of action of RP2000 is shorter than that of RP1000, suitable dosing for RP1000 will be about twice to three times greater than for RP1000. For example, suitable doses for RP2000 may include, but are not limited to, about 0.16 mg (on a cation basis) per kg of body weight to about 0.60 mg/kg RP2000 may be administered to a patient. Other contemplated dosage ranges include about 0.16 mg/kg to about 0.60 mg/kg, about 0.16 mg/kg to about 0.50 mg/kg, about 0.16 mg/kg to about 0.40 mg/kg, or about 0.24 mg/kg to about 0.45 mg/kg RP2000. Specific dosages include any there between, such as, but not limited to, about 0.16 mg/kg, about 0.24 mg/kg, about 0.32 mg/kg, about 0.40 mg/kg, and about 0.50 mg/kg RP2000. The patient may be under inhaled anesthesia of any type mentioned above.

Like RP1000, RP2000 has a relatively rapid onset period, providing an additional opportunity to minimize the time a patient is under NMB. Therefore, optionally and additionally, the administration of RP1000 to a patient during the intra-anesthetic period may effect a measured twitch height in the patient of not more than about 5% above a baseline measurement within about 2 minutes, more preferably, within about 90 seconds after administration begins.

In one or more embodiments, spontaneous recovery is achieved about 25% faster for RP2000 than for RP1000. For example, in various embodiments, spontaneous recovery may be achieved in not more than about 17 minutes after RP2000 administration is discontinued. More preferably, spontaneous recovery is achieved in not more than about 12 minutes, not more than about 10 minutes or not more than about 7 minutes.

Predictable, spontaneous recovery from NMB represents a significant advantage over current methods that utilize NMBA antagonists to reverse NMB, as these antagonists tend to be unpredictable and difficult to control. By inducing NMB with RP1000 or RP2000, the duration of NMB and the time at which infusion may be discontinued to accurately dictate timing of a spontaneous recovery. In this way, administration of a NMBA antagonist may be avoided entirely and time spent under NMB post-operatively may be reduced.

RP1000, additionally, has been proven to be safe. Any time an NMBA is used, there is the possibility of blocking critical autonomic functions, such as respiration. In animal models (e.g., monkeys and cats), there was no observed ill-effects on autonomic or circulatory systems (see, e.g., Sunaga, et al. (Preclinical Pharmacology of RP1000: A Nondepolarizing Neuromuscular Blocking Drug of Intermediate Duration, Degraded and Antagonized by l-cysteine-Additional Studies of Safety and Efficacy in the Anesthetized Rhesus Monkey and Cat; Anesthesiology, 2016 Oct; 125(4); 732-743) In dogs, only very high doses of RP1000 (27 and 54 × ED₉₅) resulted in a 20% decrease in mean arterial pressure and a 20% increase in heart rate. Furthermore, RP1000 exhibited low potential for bronchoconstrictive activity or histamine release.

As described above, spontaneous recovery of a patient from NMB using RP1000 and RP2000 while under inhaled anesthesia may be highly predictable over a wide range of doses. However, it has been observed that inhaled anesthesia, such as sevoflurane, may enhance NMB (see, *e.g.,* Ye, L., et al.; Int. J. Physicol. Pathophysiol Pharmacol; 2015 7(4), 172-177). Clinical implications dictate that a lower dose of NMBA may thus be used in a patient while under an inhaled anesthetic than would be necessary for the same level of NMB, for example, under intravenous anesthesia, such as propofol. For example, while a dose of about 0.08 mg/kg to about 0.25 mg/kg RP1000 (or about 0.08 mg/kg to about 0.20 mg/kg) may be used in a patient under inhaled anesthesia, a dose of about 0.2 mg/kg to about 0.5 mg/kg may be required to achieve the same NMB effects when the same patient is under IV anesthesia.

As such, another aspect of the present disclosure provides a method of inducing NMB comprising administering RP1000 to a human patient under IV anesthesia in an amount effective to maintain a twitch height of not more than about 5% above a baseline measurement, thereby inducing NMB in the human patient; and, after a desired duration, discontinuing administration of the RP1000 to the patient, thereby effecting a spontaneous recovery of the patient from the NMB. Optionally, NMB may be induced during an intra-operative period and/or during an intra-anesthetic period.

In various embodiments, RP1000 may be administered to the human patient in an amount of about 3.0 to about 6.0 times the ED₉₅ for humans. The amount may be administered in a single IV bolus dose, multiple IV bolus doses, or may be administered as a continuous IV infusion. Administration of a single bolus of RP1000 may be carried out over a time period of about 5 seconds to about 15 seconds. Administration in this manner may be continued, as needed, (e.g., throughout an inter-operative procedure) to maintain NMB (not more than about 5% twitch compared to baseline). Alternatively, RP1000 may be administered as a slower infusion over a time period of about 1 minute to about 2 minutes or as a continuous slow infusion (e.g., spanning at least a portion of an intra-operative period).

Specific doses include, but are not limited to, about 0.24 mg (on a cation basis) per kg of body weight to about 0.48 mg/kg RP1000 may be administered to a patient. Other contemplated dosage ranges include about 0.24 mg/kg to about 0.40 mg/kg, about 0.24 mg/kg to about 0.32 mg/kg, about 0.32 mg/kg to about 0.40 mg/kg, or about 0.32 mg/kg to about 0.48 mg/kg RP1000. Specific dosages include any there between, such as, but not limited to, about 0.24 mg/kg, about 0.30 mg/kg, about 0.32 mg/kg, about 0.35 mg/kg, about 0.40 mg/kg, about 0.45 mg/kg. and about 0.48 mg/kg RP1000. The patient may be under IV anesthesia, for example, propofol, etomidate, ketamine, barbituates (e.g., thiopental and methohexital), or any combination thereof.

In one or more embodiments, spontaneous recovery is achieved about 25% faster under IV anesthesia than under inhaled anesthesia. For example, spontaneous recovery may be achieved in not more than about 38 minutes after RP1000 administration is discontinued. More preferably, spontaneous recovery is achieved in not more than about 30 minutes, not more than about 25 minutes, not more than about 22.5 minutes or not more than about 20 minutes.

RP2000 may also be utilized to induce NMB while under IV anesthesia. Therefore, as such, another aspect of the present disclosure provides a method of inducing NMB comprising administering RP2000 to a human patient under IV anesthesia in an amount effective to maintain a twitch height of not more than about 5% above a baseline measurement, thereby inducing NMB in the human patient; and, after a desired duration, discontinuing administration of the RP2000 to the patient, thereby effecting a spontaneous recovery of the patient from the NMB. Optionally, NMB may be induced during an intra-operative period and/or during an intra-anesthetic period.

In various embodiments, RP2000 may be administered to the human patient in an amount of about 3.0 to about 6.0 times the ED₉₅ for humans. The amount may be administered in a single IV bolus dose, multiple IV bolus doses, or may be administered as a continuous IV infusion. Administration of a bolus of RP2000 may be carried out over a time period of about 5 seconds to about 15 seconds or as a slower infusion over a time period of about 1 minute to about 2 minutes. Administration in this manner may be continued, as needed, throughout an inter-operative procedure to maintain NMB (not more than about 5% twitch compared to baseline). Suitable doses include about 0.48 mg (on a cation basis) per kg of body weight to about 3.6 mg/kg RP2000 may be administered to a patient. Other contemplated dosage ranges include about 0.48 mg/kg to about 3.0 mg/kg, about 0.48 mg/kg to about 2.4 mg/kg, about 0.48 mg/kg to about 1.8 mg/kg, about 0.48 mg/kg to about 1.0 mg/kg, about 0.64 mg/kg to about 3.0 mg/kg, about 0.80 mg/kg to about 3.0 mg/kg, about 0.96 mg/kg to about 1.8 mg/kg, and about 0.96 mg/kg to about 2.4 mg/kg RP2000. Specific dosages include any there between, such as, but not limited to, 0.64 mg/kg, 0.80 mg/kg, 0.96 mg/kg, 1.80 mg/kg, 2.4 mg/kg, 3.0 mg/kg, and 3.60 mg/kg RP1000. The patient may be under IV anesthesia as described above.

In one or more embodiments, spontaneous recovery is achieved about 25% faster for RP2000 than the recovery for RP1000. For example, spontaneous may be achieved in not more than about 15 minutes after RP2000 administration is discontinued. More preferably, spontaneous recovery is achieved in not more than about 10 minutes, not more than about 7.5 minutes or not more than about 5 minutes.

While not wishing to be bound by theory, the predictability of spontaneous recovery from NMBA is thought to be derived from its metabolism in the human body, for example, by glutathione, which is readily available in the human body. This is unique to RP1000 and RP2000, as other NMBAs undergo a more complex degradation and therefore do not yield a predictable timetable for spontaneous recovery. Comparative metabolic studies have demonstrated that glutathione metabolism patterns may be specific to humans when compared to other species, including primates, therefore, data illustrating that predictability of spontaneous recovery can be achieved in humans after administration of RP1000 was particularly encouraging. Additionally, careful consideration must usually be given when administering an NMBA to a patient with disease conditions that may impact the extent of NMB as well as the decay of the NMBA in the body. Since RP1000 and RP1000 and RP2000 simply rely on glutathione for predictable decay, these agents may be used across a widely varying population, even in those with disease states or conditions that make use of other NMBAs difficult.

While predicting spontaneous recovery from NMB induced by RP1000 and RP2000 has been provided by the methods disclosed herein, advantageously, each of RP1000 and RP2000 are both particularly responsive to its respective antagonists. Reversal agents of RP1000 and RP2000 have been developed that can effectively remove the NMB caused by RP1000 within a few minutes, even when a dose three times the ED₉₅ is used. Therefore, should a situation arise wherein a patient requires immediate recovery from NMB induced by RP1000 or RP2000 and the timing of spontaneous recovery is inadequate, an antagonist of the NMB may be administered to rapidly reverse the NMB. Such agents include, but are not limited to, cysteine, glutathione, N-acetyl cysteine, homocysteine, methionine, S-adenosyl-methionine, penicillamine, a related cysteine analog, a combination thereof or a pharmaceutically acceptable salt thereof. The use of such antagonists is also disclosed in US Patent 8,148,398. In some embodiments, the antagonist is cysteine. In other embodiments, the antagonist is cysteine combined with glutathione. In other embodiments, the antagonist is cysteine or glutathione combined with any of the other antagonists. For example, in some embodiments, the combination of cysteine and glutathione is particularly effective.

RP1000 may be administered to a patient in a composition comprising RP1000. Likewise, RP2000 may be administered in a composition comprising RP2000. Compositions suitable for the methods disclosed herein comprise RP1000 or RP2000 and may be an aqueous or non-aqueous solution or a mixture of liquids, which may contain bacteriostatic agents (e.g., benzyl alcohol), antioxidants, buffers or other pharmaceutically acceptable additives (e.g., dextrose). Solvents such as alcohol, polyethylene glycol, dimethyl sulfoxide, or any mixture thereof may be included in the composition.

The composition of RP1000 or RP2000 may be administered to the human patient under inhaled anesthesia at doses as described above. For example, a suitable dose of RP1000 to obtain NMB in an adult humans (150 lbs. or 70 kg) is about 0.1 mg to about 14 mg, or in some embodiments about 1 mg to about 14 mg, or in other embodiments about 0.5 mg to about 14 mg, or in further embodiments about 3.5 mg to about 14 mg. For a human patient having a higher body weight, this dose would be greater, for example, up to about 18 mg for a 200 lb. (90 kg) patient or about 23 mg for a 250 lb. (114 kg) patient. Thus, a suitable pharmaceutical parenteral preparation for administration to humans may contain about 0.1 mg/mL to about 50 mg/mL of RP1000 in solution or multiples thereof for multi-dose vials. A similar calculation may be carried out for dosing of RP2000 based on the above disclosure.

For information, a kit may include, separately packaged, (a) RP1000 or RP2000 in an amount sufficient to relax or block skeletal muscle activity, and with (b) instructions explaining how to administer the RP1000 or RP2000 agent to a human patient. Optionally, a kit can additionally comprise (c) an amount of a RP1000 or RP2000 antagonist effective to reverse the effects of RP1000 or RP2000, respectively in a human, if needed as well as (d) instructions of how to employ the antagonist to reverse the effects of the blocking agent on the human patient to which RP1000 or RP2000 was administered. In such a kit, the RP1000 or RP2000 may be supplied in an aqueous or non-aqueous solution or a mixture of liquids, which may contain bacteriostatic agents (e.g., benzyl alcohol), antioxidants, buffers or other pharmaceutically acceptable additives (e.g., dextrose). Solvents such as alcohol, polyethylene glycol, dimethyl sulfoxide, or any mixture thereof may be included in the composition. Alternatively, the RP1000 or RP2000 may be presented in the form of a lyophilized solid, optionally with other solids, for reconstitution with water (for injection) or dextrose or saline solutions. Such formulations are normally presented in unit dosage forms such as ampoules or disposable injection devices. They may also be presented in multi-dose forms such as a bottle from which the appropriate dose may be withdrawn. All such formulations should be sterile.

For information, a method of predicting spontaneous recovery in a patient being administered an NMBA comprises subjecting the patient to TOF monitoring thereby generating electronic data comprising twitch height measurements; conveying the data to a data processing apparatus programmed to the twitch height measurements to a baseline measurement; initiate a prediction calculation at a first time defined as the time at which a twitch height measurement greater than 5% of the baseline measurement is collected; and generating a predicted spontaneous time of NMB recovery for the patient based inputting that time into a pre-programmed equation based on the spontaneous recovery times described herein.

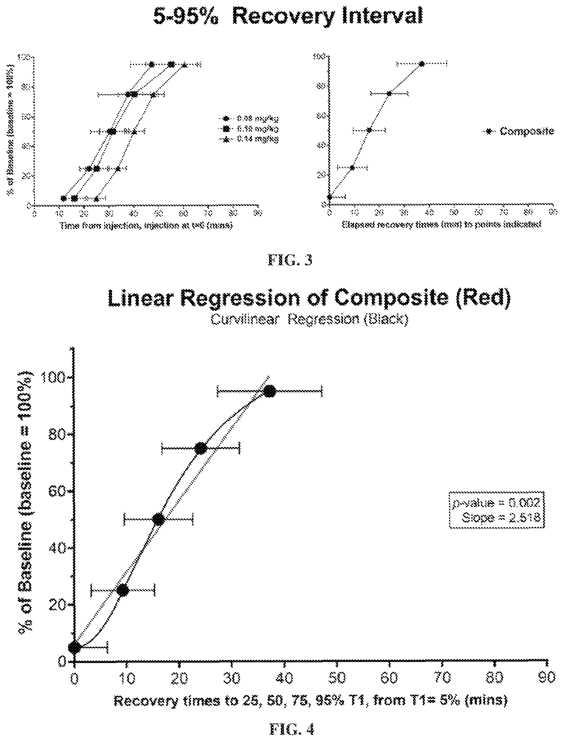

For example, for a patient under inhaled anesthesia receiving RP1000 at a dose of about 0.08 to about 0.14 mg/kg (not claimed), the time to particular twitch height above baseline may be calculated by equation (1), which is based on the data in FIG. 4, where T_{recovery} is the time to the particular twitch height, in minutes, and Ht is the particular twitch height: ${T}_{recovery} = \frac{{H}_{t} - 5}{2.52}$

The predicted recovery time may then inform if any action should be taken to ensure desired maintained of NMB with respect to anesthesia administration and intra-operative durations. For example, the calculation may alert whether further NMBA dosing is required during an intra-operative period or may inform when anesthesia may be discontinued (as NMB recovery should be achieved before recovery from the anesthesia).

Though equation 1 above pertains to the use of RP1000 under inhaled anesthesia, a similar calculation may be made for RP1000 under IV anesthesia, as well as RP2000 under either anesthesia types.

Various tests for measuring NMB are disclosed herein and described below in further detail.

Twitch Height: A peripheral nerve stimulator that applies supramaximal stimuli to the ulnar nerve at the wrist via surface electrodes was used for neuromuscular monitoring. Following anesthesia induction, single twitch stimuli (0.10 Hz) may be delivered continuously during a 15- to 20- minute period to establish baseline twitch height. Single twitch monitoring may continue during and following NMBA administration.

Train-of-Four twitch stimulation pattern ratio (TOFR): The TOF delivers 4 supramaximal electrical impulses that involve four equally strong twitches of the stimulated muscle. A fade of the twitches appears when the neuromuscular blockade increases. Comparison of the fourth twitch (T₄) to the first twitch (T₁) provides the TOFR.

Whenever a numerical range with a lower limit and an upper limit is disclosed, any number and any included range falling within the range is specifically disclosed. In particular, every range of values (of the form, "from about a to about b," or, equivalently, "from approximately a to b," or, equivalently, "from approximately a-b") disclosed herein is to be understood to set forth every number and range encompassed within the broader range of values. It must also be noted that as used herein and in the appended claims, the singular forms "a", "an", and "the" include plural reference unless the context clearly dictates otherwise. As used herein, the term "about" means plus or minus 10% of the numerical value of the number with which it is being used. Therefore, about 50% means in the range of 45%-55%.

One or more illustrative embodiments are presented herein. Not all features of a physical implementation are described or shown in this application for the sake of clarity. It is understood that in the development of a physical embodiment of the present disclosure, numerous implementation-specific decisions must be made to achieve the developer's goals, such as compliance with system-related, business-related, government-related and other constraints, which vary by implementation and from time to time. While a developer's efforts might be time-consuming, such efforts would be, nevertheless, a routine undertaking for one of ordinary skill in the art and having benefit of this disclosure.

### EXAMPLES

**Example 1: Preclinical Results in Rhesus Monkeys.** Rhesus monkeys under isoflurane anesthesia were administered a bolus dose of either RP2000 or RP1000 at a dose equal to 1x to 10x the ED₉₅ in Rhesus monkeys of the administered compound (ED₉₅, _{RP1000} = 0.040 mg/kg; ED_{95, RP2000} = 0.053 mg/kg). Twitch (0.15 Hz) and TOF (2 Hz x 2 seconds) were recorded throughout the 6 - 10 hours experiments. Time to spontaneous recovery after bolus administration, characterized by recovery of twitch from 5% to 95% of baseline was measured.

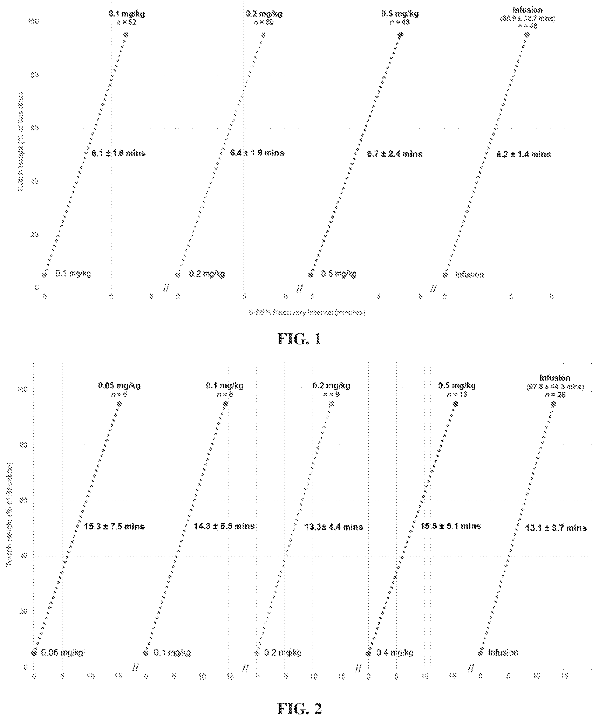

Separately, continuous infusions of 20 - 180 minutes duration were given (to separate subjects), and after discontinuation of infusion, time to spontaneous recovery of NMB, characterized by recovery of twitch from 5% to 95% of baseline was measured. Table 1 below reports the data collected from these experiments. FIG. 1 and FIG. 2 represent this data graphically.

| RP2000 | | RP1000 | |
|---|---|---|---|
| Dose (mg/kg) | 5%-95% Interval (min. ± SD) | Dose (mg/kg) | 5%-95% Interval (min. ± SD) |
| 0.10 (n = 52) | 6.1 ± 1.6 | 0.05 (n = 6)* | 15.3 ± 7.5 |
| 0.20 (n = 80) | 6.4 ± 1.9 | 0.10 (n = 8) | 14.3 ± 6.5 |
| 0.50 (n = 48) | 6.7 ± 2.4 | 0.20 (n = 9) | 13.3 ± 4.4 |
| Infusion (n = 48) | 6.2 ± 1.4 | 0.40 (n = 13) | 15.5 ± 5.1 |
| | | Infusion (n = 26) | 13.1 ± 3.7 |

The details of this experiment can be found in an abstract pertaining to Poster number F1004 at the October 2019 Anesthesiology annual meeting, the contents of which are incorporated herein by reference.

**Example 2: Phase I Clinical Trial Results in Humans.** (Abstract available in the final supplement of the Journal of Anesthesia and Analgesia, May 2020 (Volume 30, Issue 5, pages 73-74), which is incorporated herein by reference. Healthy volunteers (n=34) aged 18 to 55 of either gender gave informed consent to an IRB-approved Phase I protocol. NMB was measured by mechanomyography during sevoflurane (0.5 MAC)/N₂O (70%) anesthesia. Each volunteer received a single IV bolus of RP1000.

Table 3 below provides various pharmacokinetic data for each of the doses tested.

| **Parameter Mean (SD)/ Geometric Mean** | **0.04 mg/kg (n=6)** | **0.06 mg/kg (n=6)** | **0.08 mg/kg (n=6)** | **0.10 mg/kg (n=6)** | **0.14 mg/kg (n=4)** |
|---|---|---|---|---|---|
| Cₘₐₓ (ng/mL) | 2535 (995.24)/ 2360 | 3072 (1502.86)/ 2600 | 4041 (1948.74)/ 3610 | 5270 (1631.90)/ 5020 | 10251 (5059.49)/ 9120 |
| AUCₗₐₛₜ (min·ng/mL) | 381 (628.81)/ 7900 | 9176 (1475.55)/ 9610 | 14090 (2109.08)/ 14900 | 17431 (1699.12)/ 18200 | 28873 (4620.44)/ 30400 |
| t_{1/2} (min) | 29 (31.5)/ 27.4 | 26 (2.13)/ 26.3 | 27 (2.14)/ 27.0 | 24 (2.44)/ 23.9 | 25 (1.06)/ 24.8 |
| CL (mL/min) | 400 (55.91)/ 387 | 409 (109.14)/ 399 | 390 (51.47)/ 387 | 443 (66.93)/ 438 | 368 (41.93)/ 366 |
| Vₛₛ (mL) | 9541 (2107.50)/ 9330 | 8919 (1537.42)/ 8800 | 9651 (1305.29)/ 9580 | 9123 (1472.39)/ 9020 | 7965 (1614.98)/ 7840 |
| AUC = area under the plasma concentration-time curve; CL = clearance; Cₘₐₓ = maximum plasma concentration; SD = standard deviation; t_{1/2} = terminal elimination half-life; Tₘₐₓ = time when Cₘₐₓ occurs; Vₛₛ = volume at steady state | | | | | |

Injection of RP1000 demonstrated rapid onset of action and an intermediate duration of NMB effect. An ED₉₅ dose of 0.08 mg/kg was established under sevoflurane anesthesia and a dose of 0.14 mg/kg led to 100% twitch suppression in all subjects. At doses below 0.08 mg/kg, (n=18) 100% twitch inhibition occurred in NONE of the volunteers. 12 of 14 volunteers, however, who were given doses of 0.08, 0.10, or 0.14 mg/kg, developed 100% block: (n=2 of 6 at 0.08, 6 of 6 at 0.10, and 4 of 4 at 0.14 mg/kg). At doses ≥ ED_{95,} 95% T1 suppression was achieved in approximately 2-3 minutes, and maximum T1 suppression was achieved in approximately 3-5 minutes.

During spontaneous recovery from 100% block, in all volunteers, TOF stimulation was applied to the ulnar nerve every 20 seconds and the response of the thumb was monitored continuously until T₁ of TOF had recovered to 95% of baseline and TOFR had reached 0.90. The total duration of block was calculated from injection to recovery of T1 to 95 percent of baseline and to recovery of TOFR to 0.90. The 5-95% recovery intervals during recovery from 100% block were measured. Following the completion of data acquisition for all dosage groups, a comparison was made by ANOVA of the 5-95% recovery times resulting after doses of 0.08, 0.10 and 0.14 mg/kg. Recovery data in the 12 volunteers who developed 100% block were then combined to show a single composite recovery pattern. ANOVA was again done to compare the recovery data (5-95% intervals) for the composite group to the corresponding intervals for the separate dosage groups of 0.08, 0.10, and 0.14 mg/kg.

When allowed to spontaneously recover, mean time to 95% T₁ recovery was approximately 45, 55, and 60 minutes at the 0.08 mg/kg, 0.10 mg/kg, and 0.14 mg/kg doses, respectively. Time to maximum T₁ recovery was about 50 minutes at the 0.08 mg/kg dose and about 20 minutes longer (about 70 minutes) at 0.10 mg/kg and 0.14 mg/kg doses. The mean time to T4:T1 > 0.9 was approximately 50 minutes, 70 minutes, and 80 minutes, respectively at the 0.08 mg/kg, 0.10 mg/kg, and 0.14 mg/kg doses. The mean time from 5% to 95% T1 recovery was similar at 0.1 mg/kg and 0.145 mg/kg doses (35-40 minutes), as shown below in Table 2.

**Table 2**

| | **Doses (mg/kg)** | | | |
|---|---|---|---|---|
| | **0.08** | **0.1** | **0.14** | **Composite** |
| Recovery Interval | Recovery Time (min) | | | |
| 5%-95% | 34.8 ± 12.5 | 39.4 ± 6.5 | 35.6 ± 3.1 | 37.4 ± 6.4 |
| 25%-75% | 15.7 ± 8.4 | 15.1 ± 2.0 | 14.2 ± 0.9 | 14.9 ± 3.0 |

The recovery data for the composite group of twelve was then analyzed by linear regression. The regression essentially comprised data for 5-95% recovery time. The slope of the composite regression line was calculated. Results are summarized in Figs. 3 and 4 Fig. 3 shows apparent parallelism of all recovery curves: for groups 0.08, 0.10, and 0.14 mg/kg, and for the composite group. Both comparisons where ANOVA was applied twice and showed no significant differences among the groups 0.08, 0.10, and 0.14 mg/ kg; when comparison of the composite group was added, differences remained nonsignificant: P= 0.58 and P = 0.76 respectively. Fig. 4 shows the regression of the composite recovery line from 5% twitch height to 25, 50, 75, and 95% twitch height, versus time for the twelve individuals who developed 100% block of twitch. The relation is significant (P = 0.002). The slope of the line is 2.518.

Based on extrapolations and indirect comparisons from published human data comparing the ED₉₅ of RP1000 under sevoflurane (0.0-7 mg/kg to 0.08 mg/kg) to other marketed NMBAs, it is expected that RP1000 will have a potency about 2/3^{rds} that of cisatracurium and 4 times the potency of rocuronium under volatile anesthesia. Additionally, utilizing the same times of comparisons, the duration of NMB is expected to be about 80% to 85% that of cisatracurium and 60^ to 70% that of rocuronium. Based on data from the previous completed first human study and animal data on the onset of block of 2 times the ED_{95,} onset with RP1000 was noted to be faster than the onset achieved with cisatracurium, but slightly slower than the onset of rocuronium.

Safety. In humans, administration RP1000 in doses up to 0.14 mg/kg did not result in significant cardiopulmonary side effects nor any signs of histamine release. In general, doses of RP1000 ranging from 0.02 mg/kg to 0.14 mg/kg were generally well-tolerated among healthy volunteers enrolled in the study.

**Example 3: Metabolism of RP1000 and RP2000**. Consistent with the observations of predictable recovery times, pharmacokinetic measurements revealed that the half-life of elimination in this dosing range is also consistent at about 25-26 minutes in all dosage groups.

While not wishing to be bound by theory, it is believed that the highly reproducible half-life is due to degradation of RP1000 through cysteine adduction (e.g., by reaction with glutathione). The advantages imparted by the understanding of the pharmacodynamics of RP1000 include, but are not limited to, that the level of recovery of function may be easily predicted in human patients. Further, while not wishing to be bound by theory, it is predicted that since RP2000 is degraded in the body through similar pathways as RP1000, that spontaneous recovery of patients under inhaled anesthesia after RP2000 administration of doses up to 2.5 - 3 x the ED₉₅ will also be highly predictable as the half-life of elimination in this dosing range will be dependent on its degradation pathway. This disclosure therefore reflects this prediction.

**Example 4.** Healthy volunteers receive infusions of up to 0.24 mg/kg of RP1000 administered via IV over a period of 10 minutes while the patient is under inhaled anesthesia, IV anesthesia, or a combination thereof. Doses may include 0.8 mg/kg, 0.10 mg/kg, 0.12 mg/kg, 0.14 mg/kg, 0.16 mg/kg, 0.18 mg/kg, 0.2 mg/kg, 0.22 mg/kg, or 0.24 mg/kg. Volume of distribution of the central compartment (V_{c}) and rate constant (kₑₒ) describing the delay between plasma concentration and NMB are determined for each dose.

**Example 5.** Healthy volunteers receive either a single IV bolus of RP1000 or two single boluses of RP1000. Bolus doses may be 0.02 mg/kg, 0.4 mg/kg, 0.8 mg/kg, 0.10 mg/kg, 0.14 mg/kg, 0.16 mg/kg, 0.18 mg/kg, or 0.2 mg/kg while the patient is under inhaled anesthesia, IV anesthesia, or a combination thereof. Doses may include 0.8 mg/kg, 0.10 mg/kg, 0.12 mg/kg, 0.14 mg/kg, 0.16 mg/kg, 0.18 mg/kg, 0.2 mg/kg, 0.22 mg/kg, or 0.24 mg/kg.

## Claims

1. A compound for use in a method of inducing paralysis or neuromuscular blockade (NMB) and recovery therefrom:
wherein the method comprises administration of an effective amount of the compound to a human patient under anesthesia;
wherein the compound is selected from RP2000 and RP1000;
wherein RP2000 is 4-(3-(((E)-4-(3-((1R)-6,7-dimethoxy-1-(4-methoxybenzyl)-2-methyl-1,2,3,4-tetrahydro-2-isoquinolin-2-ium-2-yl)propoxy)-4-oxobut-2-enoyl)oxy)propyl)-4-(3,4-dimethoxybenzyl)morpholin-4-ium dichloride or any other pharmaceutically acceptable salt thereof; and
wherein RP1000 is (2S)-1-(3,4-dimethoxybenzyl)-2-(3-(((E)-4-(3-((1R,2S)-1-(3,4-dimethoxybenzyl)-6,7-dimethoxy-2-methyl-1,2,3,4-tetrahydroisoquinolin-2-ium-2-yl)propoxy)-4-oxobut-2-enoyl)oxy)propyl)-6,7-dimethoxy-2-methyl-1,2,3,4-tetrahydroisoquinolin-2-ium dichloride or any other pharmaceutically acceptable salt thereof;
wherein the effective amount of the compound RP2000 is selected from:
0.16 mg on a cation basis per kg of body weight to 0.60 mg/kg for the human patient; and
0.48 mg on a cation basis per kg of body weight to 3.6 mg/kg for the human patient;
wherein the effective amount of the compound RP1000 is selected from:
0.15 mg on a cation basis per kg of body weight to 0.25 mg/kg for the human patient; and
0.24 mg on a cation basis per kg of body weight to 0.48 mg/kg for the human patient;
wherein recovery is effected by discontinuation of administration of the compound and is **characterized by** the measurement of a train-of-four (TOF) ratio of any least 0.90 in the human patient.

2. The compound for use of claim 1:
wherein the effective amount of the compound RP2000 is selected from:
0.16 mg on a cation basis per kg of body weight to 0.60 mg/kg for the human patient when the anesthesia is inhaled anesthesia; and
0.48 mg on a cation basis per kg of body weight to 3.6 mg/kg for the human patient when the anesthesia is intravenous (IV) anesthesia;
wherein the effective amount of the compound RP1000 is selected from:
0.15 mg on a cation basis per kg of body weight to 0.25 mg/kg for the human patient when the anesthesia is inhaled anesthesia; and
0.24 mg on a cation basis per kg of body weight to 0.48 mg/kg for the human patient when the anesthesia is intravenous (IV) anesthesia.

3. The compound for use of claim 1 or claim 2 wherein the compound is RP2000 and the method comprises inducing paralysis or neuromuscular blockade (NMB) and recovery therefrom comprising:
administration of the effective amount of RP2000 to the human patient under anesthesia; and
effecting a spontaneous recovery from the paralysis or NMB in the absence of an RP2000 antagonist, wherein the spontaneous recovery is **characterized by** the measurement of a TOF ratio of at least 0.90 in the human patient.

4. The compound for use of claim 3, wherein the anesthesia is inhaled anesthesia.

5. The compound for use of claim 3, wherein the anesthesia is IV anesthesia.

6. The compound for use of claim 5, wherein the effective amount of RP2000 is 0.48 mg/kg to 2.00 mg/kg.

7. The compound for use of claim 1 or claim 2 wherein the compound is RP1000 and the method comprises inducing paralysis or neuromuscular blockade (NMB) and recovery therefrom comprising:
administration of the effective amount of RP1000 to the human patient under anesthesia; and
effecting a spontaneous recovery from the paralysis or NMB in the absence of an RP1000 antagonist, wherein the spontaneous recovery is **characterized by** the measurement of a TOF ratio of at least 0.90 in the human patient.

8. The compound for use of claim 7, wherein the anesthesia is inhaled anesthesia.

9. The compound for use of claim 7, wherein the anesthesia is IV anesthesia.

10. The compound for use of claim 9, wherein the effective amount of RP1000 is 0.24 mg on a cation basis per kg of body weight to 0.48 mg/kg.

11. A pharmaceutical composition for use of any one of claims 1 to 10, comprising the compound for use of any one of claims 1 to 10, wherein the pharmaceutical composition optionally further comprises a solvent selected from one or more of an alcohol, polyethylene glycol, and dimethyl sulfoxide.

## Patentansprüche

1. Verbindung zur Anwendung bei einem Verfahren zum Induzieren von Paralyse oder neuromuskulärer Blockade (NMB) und deren Erholung:
wobei das Verfahren die Verabreichung einer wirksamen Menge der Verbindung an einen menschlichen Patienten unter Anästhesie umfasst;
wobei die Verbindung ausgewählt ist aus RP2000 und RP1000;
wobei RP2000 4-(3-(((E)-4-(3-((1R)-6,7-Dimethoxy-1-(4-methoxybenzyl)-2-methyl-1,2,3,4-tetrahydro-2-isochinolin-2-ium-2-yl)propoxy)-4-oxobut-2-enoyl)oxy)propyl)-4-(3,4-dimethoxybenzyl)morpholin-4-iumdichlorid oder ein beliebiges anderes pharmazeutisch verträgliches Salz davon ist; und
wobei RP1000 (2S)-1-(3,4-Dimethoxybenzyl)-2-(3-(((E)-4-(3-((1R,2S)-1-(3,4-dimethoxybenzyl)-6,7-dimethoxy-2-methyl-1,2,3,4-tetrahydroisochinolin-2-ium-2-yl)propoxy)-4-oxobut-2-enoyl)oxy)propyl)-6,7-dimethoxy-2-methyl-1,2,3,4-tetrahydroisochinolin-2-iumdichlorid oder ein beliebiges anderes pharmazeutisch verträgliches Salz davon ist;
wobei die wirksame Menge der Verbindung RP2000 ausgewählt ist aus:
0,16 mg auf Kationenbasis pro kg Körpergewicht bis 0,60 mg/kg für den menschlichen Patienten; und
0,48 mg auf Kationenbasis pro kg Körpergewicht bis 3,6 mg/kg für den menschlichen Patienten;
wobei die wirksame Menge der Verbindung RP1000 ausgewählt ist aus:
0,15 mg auf Kationenbasis pro kg Körpergewicht bis 0,25 mg/kg für den menschlichen Patienten; und
0,24 mg auf Kationenbasis pro kg Körpergewicht bis 0,48 mg/kg für den menschlichen Patienten;
wobei die Erholung durch Absetzen der Verabreichung der Verbindung bewirkt wird und durch die Messung eines Train-of-Four(TOF)-Verhältnisses von mindestens 0,90 bei dem menschlichen Patienten gekennzeichnet ist.

2. Verbindung zur Anwendung nach Anspruch 1:
wobei die wirksame Menge der Verbindung RP2000 ausgewählt ist aus:
0,16 mg auf Kationenbasis pro kg Körpergewicht bis 0,60 mg/kg für den menschlichen Patienten, wenn die Anästhesie eine Inhalationsanästhesie ist; und
0,48 mg auf Kationenbasis pro kg Körpergewicht bis 3,6 mg/kg für den
menschlichen Patienten, wenn die Anästhesie eine intravenöse (IV) Anästhesie ist; wobei die wirksame Menge der Verbindung RP1000 ausgewählt ist aus:
0,15 mg auf Kationenbasis pro kg Körpergewicht bis 0,25 mg/kg für den menschlichen Patienten, wenn die Anästhesie eine Inhalationsanästhesie ist; und
0,24 mg auf Kationenbasis pro kg Körpergewicht bis 0,48 mg/kg für den menschlichen Patienten, wenn die Anästhesie eine intravenöse (IV) Anästhesie ist.

3. Verbindung zur Anwendung nach Anspruch 1 oder Anspruch 2, wobei die Verbindung RP2000 ist und das Verfahren das Induzieren von Paralyse oder neuromuskulärer Blockade (NMB) und deren Erholung umfasst, umfassend:
Verabreichung der wirksamen Menge von RP2000 an den menschlichen Patienten unter Anästhesie; und
Bewirken einer spontanen Erholung von der Paralyse oder NMB in der Abwesenheit eines RP2000-Antagonisten, wobei die spontane Erholung durch die Messung eines TOF-Verhältnisses von mindestens 0,90 bei dem menschlichen Patienten gekennzeichnet ist.

4. Verbindung zur Anwendung nach Anspruch 3, wobei die Anästhesie eine Inhalationsanästhesie ist.

5. Verbindung zur Anwendung nach Anspruch 3, wobei die Anästhesie eine IV-Anästhesie ist.

6. Verbindung zur Anwendung nach Anspruch 5, wobei die wirksame Menge von RP2000 0,48 mg/kg bis 2,00 mg/kg beträgt.

7. Verbindung zur Anwendung nach Anspruch 1 oder Anspruch 2, wobei die Verbindung RP1000 ist und das Verfahren das Induzieren von Paralyse oder neuromuskulärer Blockade (NMB) und deren Erholung umfasst, umfassend:
Verabreichung der wirksamen Menge von RP1000 an den menschlichen Patienten unter Anästhesie; und
Bewirken einer spontanen Erholung von der Paralyse oder NMB in der Abwesenheit eines RP1000-Antagonisten, wobei die spontane Erholung durch die Messung eines TOF-Verhältnisses von mindestens 0,90 bei dem menschlichen Patienten gekennzeichnet ist.

8. Verbindung zur Anwendung nach Anspruch 7, wobei die Anästhesie eine Inhalationsanästhesie ist.

9. Verbindung zur Anwendung nach Anspruch 7, wobei die Anästhesie eine IV-Anästhesie ist.

10. Verbindung zur Anwendung nach Anspruch 9, wobei die wirksame Menge von RP1000 0,24 mg auf Kationenbasis pro kg Körpergewicht bis 0,48 mg/kg beträgt.

11. Pharmazeutische Zusammensetzung zur Anwendung nach einem der Ansprüche 1 bis 10, umfassend die Verbindung zur Anwendung nach einem der Ansprüche 1 bis 10, wobei die pharmazeutische Zusammensetzung optional ferner ein Lösungsmittel umfasst, das ausgewählt ist aus einem oder mehreren von einem Alkohol, Polyethylenglykol und Dimethylsulfoxid.

## Revendications

1. Composé destiné à être utilisé dans une méthode d'induction d'une paralysie ou d'un blocage neuromusculaire (NMB) et de récupération de ceux :
dans lequel la méthode comprend l'administration d'une quantité efficace du composé à un patient humain sous anesthésie ;
dans lequel le composé est choisi parmi RP2000 et RP1000 ;
dans lequel RP2000 est le dichlorure de 4-(3-(((E)-4-(3-((1R)-6,7-diméthoxy-1-(4-méthoxybenzyl)-2-méthyl-1,2,3,4-tétrahydro-2-isoquinolin-2-ium-2-yl)propoxy)-4-oxobut-2-énoyl)oxy)propyl)-4-(3,4-diméthoxybenzyl)morpholin-4-ium ou tout autre sel pharmaceutiquement acceptable de celui-ci ; et
dans lequel RP1000 est le dichlorure de (2S)-1-(3,4-diméthoxybenzyl)-2-(3-(((E)-4-(3-((1R,2S)-1-(3,4-diméthoxybenzyl)-6,7-diméthoxy-2-méthyl-1,2,3,4-tétrahydroisoquinolin-2-ium-2-yl)propoxy)-4-oxobut-2-énoyl)oxy)propyl)-6,7-diméthoxy-2-méthyl-1,2,3,4-tétrahydroisoquinolin-2-ium ou tout autre sel pharmaceutiquement acceptable de celui-ci ;
dans lequel la quantité efficace du composé RP2000 est choisie parmi :
0,16 mg sur une base cationique par kg de poids corporel à 0,60 mg/kg pour le patient humain ; et
0,48 mg sur une base cationique par kg de poids corporel à 3,6 mg/kg pour le patient humain ;
dans lequel la quantité efficace du composé RP1000 est choisie parmi :
0,15 mg sur une base cationique par kg de poids corporel à 0,25 mg/kg pour le patient humain ; et
0,24 mg sur une base cationique par kg de poids corporel à 0,48 mg/kg pour le patient humain ;
dans lequel la récupération est réalisée par l'arrêt de l'administration du composé et est **caractérisée par** la mesure d'un rapport train-de-quatre (TOF) d'au moins 0,90 chez le patient humain.

2. Composé destiné à être utilisé selon la revendication 1 :
dans lequel la quantité efficace du composé RP2000 est choisie parmi :
0,16 mg sur une base cationique par kg de poids corporel à 0,60 mg/kg pour le patient humain lorsque l'anesthésie est une anesthésie inhalée ; et
0,48 mg sur une base cationique par kg de poids corporel à 3,6 mg/kg pour le patient humain lorsque l'anesthésie est une anesthésie intraveineuse (IV) ;
dans lequel la quantité efficace du composé RP1000 est choisie parmi :
0,15 mg sur une base cationique par kg de poids corporel à 0,25 mg/kg pour le patient humain lorsque l'anesthésie est une anesthésie inhalée ; et
0,24 mg sur une base cationique par kg de poids corporel à 0,48 mg/kg pour le patient humain lorsque l'anesthésie est une anesthésie intraveineuse (IV).

3. Composé destiné à être utilisé selon la revendication 1 ou la revendication 2, dans lequel le composé est RP2000 et la méthode comprend l'induction d'une paralysie ou d'un blocage neuromusculaire (NMB) et la récupération de ceux comprenant :
l'administration de la quantité efficace de RP2000 au patient humain sous anesthésie ; et
la réalisation d'une récupération spontanée de la paralysie ou du NMB en l'absence d'un antagoniste de RP2000, dans lequel la récupération spontanée est **caractérisée par** la mesure d'un rapport TOF d'au moins 0,90 chez le patient humain.

4. Composé destiné à être utilisé selon la revendication 3, dans lequel l'anesthésie est une anesthésie inhalée.

5. Composé destiné à être utilisé selon la revendication 3, dans lequel l'anesthésie est une anesthésie IV.

6. Composé destiné à être utilisé selon la revendication 5, dans lequel la quantité efficace de RP2000 est de 0,48 mg/kg à 2,00 mg/kg.

7. Composé destiné à être utilisé selon la revendication 1 ou la revendication 2, dans lequel le composé est RP1000 et la méthode comprend l'induction d'une paralysie ou d'un blocage neuromusculaire (NMB) et la récupération de ceux comprenant :
l'administration de la quantité efficace de RP1000 au patient humain sous anesthésie ; et
la réalisation d'une récupération spontanée de la paralysie ou du NMB en l'absence d'un antagoniste de RP1000, dans lequel la récupération spontanée est **caractérisée par** la mesure d'un rapport TOF d'au moins 0,90 chez le patient humain.

8. Composé destiné à être utilisé selon la revendication 7, dans lequel l'anesthésie est une anesthésie inhalée.

9. Composé destiné à être utilisé selon la revendication 7, dans lequel l'anesthésie est une anesthésie IV.

10. Composé destiné à être utilisé selon la revendication 9, dans lequel la quantité efficace de RP1000 est de 0,24 mg sur une base cationique par kg de poids corporel à 0,48 mg/kg.

11. Composition pharmaceutique destinée à être utilisée selon l'une quelconque des revendications 1 à 10, comprenant le composé destiné à être utilisé selon l'une quelconque des revendications 1 à 10, dans lequel la composition pharmaceutique comprend en outre éventuellement un solvant choisi parmi l'un ou plusieurs d'un alcool, d'un polyéthylèneglycol et d'un diméthylsulfoxyde.
